(19) Europäisches Patentamt
European Patent Office
Office européen des brevets

(11) Veröffentlichungsnummer: **0 142 793**
**B1**

(12) # EUROPÄISCHE PATENTSCHRIFT

(45) Veröffentlichungstag der Patentschrift: **08.11.89**

(51) Int. Cl.$^4$: **C 12 N 9/18**

(21) Anmeldenummer: **84113534.6**

(22) Anmeldetag: **09.11.84**

(54) **Verfahren zur Gewinnung von Cholesterinesterase.**

(30) Priorität: **11.11.83 DE 3340950**

(43) Veröffentlichungstag der Anmeldung: **29.05.85 Patentblatt 85/22**

(45) Bekanntmachung des Hinweises auf die Patenterteilung: **08.11.89 Patentblatt 89/45**

(84) Benannte Vertragsstaaten: **AT BE CH DE FR GB IT LI LU NL SE**

(56) Entgegenhaltungen:
**DE-A- 2 933 646**
**FR-A- 2 362 927**

**CHEMICAL ABSTRACTS, Band 92, Nr. 3, 21. Januar 1980, Seite 530, Zusammenfassung Nr. 20576v, Columbus, Ohio, US; & JP-A-79 113 493**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **von der Eltz, Herbert, Dr. rer.nat., Kerschensteinerstrasse 18, D-8120 Weilheim (DE)**
Erfinder: **Gauhl, Helmgard, Mitterfeld 4, D-8132 Tutzing (DE)**
Erfinder: **Seidel, Hans, Dr. rer.nat., Waxensteinstrasse 6, D-8132 Tutzing (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Gewinnung von Cholesterinesterase aus Mikroorganismen.

Cholesterinesterase spielt eine wichtige Rolle in der klinischen und biochemischen Analytik seit Verfahren zur enzymatischen Bestimmung von Cholesterin entwickelt wurden. Da ein großer Teil des Cholesterins in biologischem Material in Form von Estern vorliegt, ermöglicht die gemeinsame Verwendung von Cholesterinesterase und Cholesterin oxidierenden Enzymen, wie Cholesterinoxidase oder Cholesterindehydrogenase, eine vollenzymatische Bestimmung auch von Cholesterinestern. Dies ist bekannt aus DE-PS 2 264 847. Als besonders geeignet hat sich im Rahmen der Cholesterin-Bestimmung dabei die Cholesterinesterase aus Mikroorganismen erwiesen (DE-OS 2 506 712.3). Die bisher aufgefundenen Cholesterinesterasebildenden Mikroorganismen benötigen nun in der Regel einen Induktor, um einen die Aufarbeitung lohnenden Gehalt an Cholesterinesterase zu bilden. Unter Induktor wird hierbei eine Substanz verstanden, welche den Mikroorganismus dazu anregt, das gewünschte Enzym in größerer Menge zu bilden als ohne Induktor. Der Induktor wird dabei dem für die Züchtung verwendeten Nährmedium zugesetzt. Bekannte Induktoren sind Fette und Öle wie Olivenöl, Sojaöl, Sonnenblumenöl, Rindertalg, Schweineöl, Erdnußöl, Fettsäureester, Cholesterinester, Palmitinsäure sowie insbesondere Lecithin (DE-OS 2 933 646), mit welchem bisher die besten Resultate bezüglich der erzielbaren Enzymmenge erreicht wurden. Jedoch wäre eine weitere Steigerung der Enzymausbeuten erwünscht. Daher besteht ein Bedarf an der Zurverfügungstellung noch besserer Induktoren.

Nunmehr wurde überraschenderweise gefunden, daß der hohe Anteil an ungesättigten Fettsäuren im Lecithin ebenso wie die Cholingruppe für die Induktorwirkung nicht optimal sind. Ausgehend von dieser Erkenntnis läßt sich die gewünschte Verbesserung daher erfindungsgemäß erreichen durch ein Verfahren zur Gewinnung von Cholesterinesterase aus Mikroorganismen durch Züchtung eines zur Cholesterinesterasebildung befähigten Mikroorganismus in einem geeigneten Nährmedium in Gegenwart eines Induktors und Gewinnung des Enzyms aus der Kulturflüssigkeit oder/und den Zellen, welches dadurch gekennzeichnet ist, daß man als Induktor eine Verbindung der allgemeinen Formel

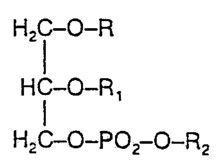

in der R und $R_1$ Alkyl- oder Alkoylgruppen mit 14 bis 18 C-Atomen, R oder $R_1$ auch ein H-Atom und $R_2$ eine Alkylamingruppe mit 2 bis 8 C-Atomen, eine Alkyl-Trimethylammoniumgruppe mit 3 bis 8 C-Atomen, eine Alkylpyridingruppe mit 1 bis 4 C-Atomen im Alkylrest oder einen Rest der Formel $-CH_2-(CHOH)_n-CH_2OH$ mit n = 1 bis 4 bedeutet, verwendet.

Erfindungsgemäß gelingt es, die Ausbeuten an Cholesterinesterase, bezogen auf gleiche Induktormengen, bis zu mehreren 100% verglichen mit Lecithin, zu verbessern. Dabei werden im Rahmen der Erfindung die besten Ergebnisse mit Verbindungen der Kephalinreihe ($R_2$ = Alkylamino) bzw. der Phosphopolyolreihe ($R_2$ = $CH_2-(CHOH)_n-CH_2OH$) und der Phosphoalkylpyridinreihe erzielt.

Weiter wurde gefunden, daß unter den gesättigten Fettsäureresten, also den Alkoylgruppen, die Stearoylgruppe eine besonders gute Wirksamkeit aufweist, insbesondere wenn sie in Position 2 des Glycerinrestes steht ($R_1$). Bei sonst identischem Aufbau des Moleküls wurde weiter gefunden, daß etwas bessere Ergebnisse erzielt werden, wenn R und $R_1$ verschieden sind, verglichen mit R = $R_1$.

Die in den Beispielen aufgeführte Tabelle 1 gibt für eine Reihe der erfindungsgemäß zu verwendenden Induktoren die unter den Bedingungen des Beispiels erzielten Enzymaktivitäten an. Zum Vergleich wurde Sojalecithin mitgeführt. Man erkennt, daß eine wesentlich überlegene Enzymausbeute mit den erfindungsgemäßen Induktoren erhalten wird. Ähnliche Ergebnisse wurden mit zahlreichen anderen der erfindungsgemäß eingesetzten Induktoren erzielt. So wurden beispielsweise sehr gute Enzymausbeuten erhalten mit
1,2-Dipalmitoyl-glycero-3-phosphoethanolamin,
1,2-Dipalmitoyl-glycero-3-phosphohexanolamin,
1,2-Dimyristoyl-glycero-3-phosphoglycerin,
1,2-Dimyristoyl-glycero-3-phosphoethanolamin und -phosphopropanolamin,
1-Palmitoyl-2-tetradecyl-glycero-3-phosphoethanolamin,
1-Octadecyl-2-tetradecyl-glycero-3-phosphoethanolamin,
1-Palmitoyl-2-stearoyl-glycero-3-phospho-trimethylammoniumpropanol und
1-Hexadecyl-glycero-3-phosphoethanolamin.

Die erfindungsgemäß eingesetzten Induktoren werden vorzugsweise auch als Kohlenstoffquelle, insbesondere als alleinige Kohlenstoffquelle für die Züchtung des Mikroorganismus verwendet. Wahlweise ist es aber auch möglich, gesonderte Kohlenstoffquellen zuzusetzen, beispielsweise Maisquellwasser, Peptone, Hefeextrakte, Zucker oder Polyalkohole.

Die beim erfindungsgemäßen Verfahren eingesetzten Induktormengen entsprechen denen der bekannten Induktoren. Sie liegen im allgemeinen zwischen etwa 0,1 und 5 Gew.-%, bezogen auf das Volumen des Nährmediums, bevorzugt zwischen 0,5 und 2 Gew.-%.

Als Mikroorganismen können im Rahmen des erfindungsgemäßen Verfahrens alle Mikroorganismen verwendet werden, welche Cholesterinesterase in einer die Aufarbeitung lohnenden Menge zu bilden vermögen. Typische Beispiele für geeignete Mikroorganismen sind in der DE-OS 2 933 646 angegeben.

Auch die sonstigen Züchtungsbedingungen können unverändert von den bekannten Verfahren, beispielsweise den oben erwähnten, übernommen werden. Dabei sind im allgemeinen die für den jeweils verwendeten Mikroorganismus günstigsten Züchtungsbedingungen wesentlich, während die Art des verwendeten Induktors normalerweise die Züchtungsbedingungen nicht beeinflußt.

Die folgenden Beispiele erläutern die Erfindung weiter:

Beispiele 1 bis 7

Zur Vorkultivierung werden 150 ml Standardmedium I der Firma Merck in einen 500 ml Erlenmeyer-Kolben gegeben und mit einer Öse voll Material des Stammes Pseudomonas spec. DSM 1280 beimpft und 15 Std. bei 28 °C geschüttelt. Die erhaltene trübe Lösung wird 1:20 mit Wasser verdünnt. Die Trübungsmessung bei 400 bis 600 nm ergab E = 0,170. 100 ml des den jeweiligen Induktor enthaltenden Mediums der unten angegebenen Zusammensetzung werden mit 1% der oben beschriebenen Vorkultur beimpft und bei 28° in einem 500 ml Erlenmeyer-Kolben geschüttelt. Das induktorhaltige Medium enthält auf 1 l Lösung folgende Substanzen:

7 g $Na_2HPO_4 \times 7H_2O$
3 g $KH_2PO_4$
1,5 g Harnstoff
0,6 g $MgSO_4 \times 7H_2O$
0,1 g $CaCl_2$
0,1 ml $FeCl_3 \times 6H_2O$ (1 g/100 ml)
0,1 ml $ZnSO_4 \times 7H_2O$ (1 g/100 ml)
50 mg NaCl
10 g Induktor

Der pH-Wert des Mediums beträgt 7,0.

Die Cholesterinesteraseaktivität wurde zu verschiedenen Zeiten in der Kultur bestimmt unter Verwendung von Cholesterinoleat; Temperatur 25 °C. Tabelle 1 zeigt die verwendete Induktoren und die nach drei Tagen erreichte Aktivität in E/L. Zum Vergleich wurde der bestwirksame bekannte Induktor, nämlich Lecithin, eingesetzt.

Tabelle 1

| Beispiel | Verbindung | E/L |
|---|---|---|
| 1 | 1,2-Dipalmitoyl-glycero-3-phosphoglycerin | 3500 |
| 2 | 1,2-Dipalmitoyl-glycero-3-phospho-3-hydroxymethyl-pyridinester | 5500 |
| 3 | 1,2-Distearoyl-glycero-3-phosphoethanolamin | 6500 |
| 4 | 1-Palmitoyl-2-stearoyl-glycero-3-phosphoethanolamin | 9000 |
| 5 | 1-Stearoyl-2-myristoyl-glycero-3-phosphoethanolamin | 5000 |
| 6 | 1-Myristoyl-2-stearoyl-glycero-3-phosphoethanolamin | 7000 |
| 7 (Vergleich) | Soja-Lecithin | 2500 |

Da alle oben angegebenen Werte unter gleichen Bedingungen erhalten wurden, sind sie exakt vergleichbar.

**Patentansprüche**

1. Verfahren zur Gewinnung von Cholesterinesterase aus Mikroorganismen durch Züchtung eines zur Cholesterinesterasebildung befähigten Mikroorganismus in einem geeigneten Nährmedium in Gegenwart eines Induktors und Gewinnung des Enzyms aus der Kulturflüssigkeit oder/und den Zellen, dadurch gekennzeichnet, daß man als Induktor eine Verbindung der allgemeinen Formel

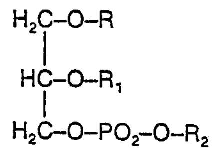

in der R und $R_1$ Alkyl- oder Alkoylgruppen mit 14 bis 18 C-Atomen, R oder $R_1$ auch ein H-Atom und $R_2$ eine Alkylamingruppe mit 2 bis 8 C-Atomen, eine Alkyl-Trimethylammoniumgruppe mit 3 bis 8 C-Atomen, eine Alkylpyridingruppe mit 1 bis 4 C-Atomen im Alkylrest oder einen Rest der Formel $-CH_2-(CHOH)_n-CH_2OH$ mit n = 1 bis 4 bedeuten, verwendet.

2. Verfahren nach Anspruch 1, dadurch gekennzeichnet, daß man eine Verbindung der allgemeinen Formel verwendet, in der $R_2$ eine Ethylaminogruppe darstellt.

3. Verfahren nach Anspruch 1 oder 2, dadurch gekennzeichnet, daß wenigstens einer der Reste R und $R_1$ eine Stearoylgruppe darstellt.

4. Verfahren nach einem der vorhergehenden Ansprüche, dadurch gekennzeichnet, daß R und $R_1$ verschieden sind.

5. Verfahren nach Anspruch 4, dadurch gekennzeichnet, daß $R_1$ eine Stearoylgruppe ist.

6. Verfahren nach einem der vorhergehenden Ansprüche, dadurch gekennzeichnet, daß $R_2$ ein Glycerinrest ist.

**Claims**

1. Process for the obtaining of cholesterol esterase from micro-organisms by culturing of a micro-organism capable of cholesterol esterase formation in a suitable nutrient medium in the presence of an inductor and obtaining of the enzyme from the culture liquid and/or the cells, characterised in that as inductor one uses a compound of the general formula:

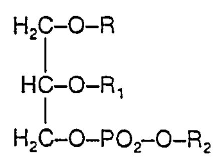

in which R and R signify alkyl or alkoyl groups with 14 to 18 C-atoms, and R or $R_1$ also an H-atom and $R_2$ is an alkylamino group with 2 to 8 C-atoms, an

alkyl-trimethylammonium group with 3 to 8 C-atoms, an alkylpyridine group with 1 to 4 C-atoms in the alkyl radical or a radical of the formula $-CH_2-(CHOH)_n-CH_2OH$ whith $n = 1$ to 4.

2. Process according to claim 1, characterised in that one uses a compound of the general formula in which $R_2$ represents an ethylamino group.

3. Process according to claim 1 or 2, characterised in that at least one of the radicals R and $R_1$ represents a stearoyl group.

4. Process according to one of the preceding claims, characterised in that R and $R_1$ are different.

5. Process according to claim 4, characterised in that $R_1$ is a stearoyl group.

6. Process according to one of the preceding claims, characterised in that $R_2$ is a glycerol radical.

**Revendications**

1. Procédé de production de cholestérol estérase à partir de microorganismes par culture d'un microorganisme apte à former de la cholestérol estérase dans un milieu nutritif approprié en présence d'un inducteur et récupération de l'enzyme à partir du liquide de culture ou/et des cellules, caractérisé en ce qu'on utilise, en tant qu'inducteur, un composé de formule générale

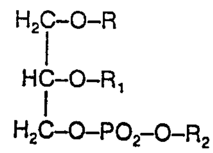

dans laquelle R et $R_1$ représentent des groupes alcoyle ou alcanoyle avec 14 à 18 atomes de C, R ou $R_1$ représente également un atome de H et $R_2$ un groupe alcoylamino avec 2 à 8 atomes de C, un groupe alcoyl-triméthylammonium avec 3 à 8 atomes de C, un groupe alcoylpyridine avec 1 à 4 atomes de C dans le radical alcoyle ou un radical de formule $-CH_2-(CHOH)_n-CH_2OH$ avec $n = 1$ à 4.

2. Procédé selon la revendication 1, caractérisé en ce qu'on utilise un composé selon la formule générale dans laquelle $R_2$ représente un groupe éthylamino.

3. Procédé selon la revendication 1 ou 2, caractérisé en ce qu'au moins l'un des radicaux R et $R_1$ représente un groupe stéaroyle.

4. Procédé selon l'une des revendications précédentes, caractérisé en ce que R et $R_1$ sont différents.

5. Procédé selon la revendication 4, caractérisé en ce que $R_1$ est un groupe stéaroyle.

6. Procédé selon l'une des revendications précédentes, caractérisé en ce que $R_2$ est un radical glycérol.